(19) Europäisches Patentamt
European Patent Office
Office européen des brevets

(11) **EP 4 703 323 A1**

(12) **EUROPEAN PATENT APPLICATION**
published in accordance with Art. 153(4) EPC


(43) Date of publication:
**04.03.2026 Bulletin 2026/10**

(21) Application number: **24796964.5**

(22) Date of filing: **22.04.2024**

(51) International Patent Classification (IPC):
***C01B 13/02*** $^{(2006.01)}$ ***A61K 33/00*** $^{(2006.01)}$
***A61P 31/04*** $^{(2006.01)}$ ***A61P 31/12*** $^{(2006.01)}$
***A61P 35/00*** $^{(2006.01)}$ ***C07D 487/22*** $^{(2006.01)}$

(52) Cooperative Patent Classification (CPC):
**A61K 33/00; A61P 31/04; A61P 31/12; A61P 35/00; C01B 13/02;** C07D 487/22

(86) International application number:
**PCT/JP2024/015705**

(87) International publication number:
**WO 2024/225218 (31.10.2024 Gazette 2024/44)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **25.04.2023 JP 2023071394**
**08.04.2024 JP 2024062199**



(71) Applicant: **Canon Kabushiki Kaisha**
**Tokyo, 146-8501 (JP)**

(72) Inventors:
- **NOMURA, Shotaro**
**Tokyo 146-8501 (JP)**
- **NISHIDA, Tsutomu**
**Tokyo 146-8501 (JP)**
- **SURUGA, Eika**
**Tokyo 146-8501 (JP)**
- **OHIRA, Kazunari**
**Tokyo 146-8501 (JP)**
- **TAMURA, Shigeto**
**Tokyo 146-8501 (JP)**
- **NAKAMOTO, Atsushi**
**Tokyo 146-8501 (JP)**
- **SAKAKIBARA, Akira**
**Tokyo 146-8501 (JP)**
- **MATSUDA, Shotaro**
**Tokyo 146-8501 (JP)**
- **TOMATSU, Fumiko**
**Tokyo 146-8501 (JP)**
- **SHICHIJO, Ibuki**
**Tokyo 146-8501 (JP)**

(74) Representative: **TBK**
**Bavariaring 4-6**
**80336 München (DE)**


(54) **METHOD FOR GENERATING SINGLET OXYGEN, SYSTEM FOR GENERATING SINGLET OXYGEN, KIT FOR GENERATING SINGLET OXYGEN, AND AGENT FOR GENERATING SINGLET OXYGEN**


(57) This method for generating singlet oxygen is characterized in that a step for generating singlet oxygen by irradiating a phtalocyanine compound crystal with electromagnetic radiation is included; an X-ray diffraction pattern that is obtained for the phtalocyanine compound crystal through X-ray diffraction measurement using CuKα ray has peaks within a range of diffraction angle 2θ = 5° to 35°; and that the full width at half maximum of the peak exhibiting the highest intensity within the range is 2.50° or less.


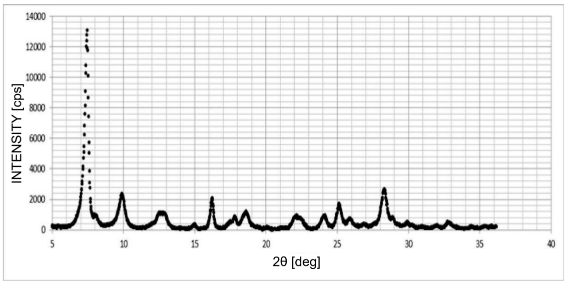


Fig. 1

EP 4 703 323 A1

## Description

### TECHNICAL FIELD

The present disclosure relates to a method for generating singlet oxygen, a system for generating singlet oxygen, a kit for generating singlet oxygen, and an agent for generating singlet oxygen.

### BACKGROUND ART

Singlet oxygen, i.e., a type of active oxygen, is used for the oxidative decomposition of organic matter, killing viruses and bacteria, and killing cancer cells in a living body capitalizing on high reactivity and oxidizing power thereof.

A method for generating singlet oxygen can be exemplified by a chemical method such as using an oxygen generator, in which excitation is provided by a chemical oxygen iodine laser and in which chlorine gas is reacted with an aqueous hydrogen peroxide solution made basic with potassium hydroxide or the like. Alternatively, a simpler method is a photochemical method, in which singlet oxygen is generated by intersystem crossing between an oxygen molecule and a colorant molecule excited to a triplet excited state by electromagnetic waves such as light.

A practical example of the latter method is photodynamic therapy, which is a cancer cell killing therapy using singlet oxygen. Specific examples of colorant molecules include porphyrin compounds and phthalocyanine compounds.

However, this conventional method for generating singlet oxygen requires visible light or infrared light to excite the colorant, hence practical application thereof is limited only to areas where irradiation with the excitation light is possible. This is particularly problematic when there is a medium that surrounds the colorant and blocks the excitation light. For example, in photodynamic therapy, the problem is how to irradiate deep zones inside the body with sufficient excitation light.

In order to solve this problem, attempts have been made to design colorant molecules that can be excited by light with longer wavelengths and that have high biological permeability.

In addition, in search of an excitation means having higher medium permeability, excitation by X-rays has also been attempted. In addition to a method of directly exciting colorant molecules, such as porphyrin, with X-rays (PTL 1), there is a method of absorbing X-rays by, for instance, heavy elements near the colorant and exciting the colorant with excitation light from the excited heavy elements (PTL 2). A method for exciting a colorant with Auger electrons generated by irradiating a heavy element with X-rays is also known (PTL 3).

### CITATION LIST

### PATENT LITERATURE

[PTL 1] Japanese Patent Laid-Open No. 2009-155239.

[PTL 2] Japanese Translation of PCT Application No. 2010-523690.

[PTL 3] Japanese Translation of PCT Application No. 2017-532340.

### SUMMARY OF INVENTION

### TECHNICAL PROBLEM

However, when visible light or infrared light is used currently as an excitation means, even if the wavelength of the excitation light is increased, it is not possible to avoid absorption by a medium such as the human body, and the amount of singlet oxygen generated in a target area does not necessarily satisfy the necessary amount required for the application.

Furthermore, when X-rays are used as an excitation means, there is a problem that there are currently no monochromatic X-ray sources that are widely used in society and are highly practical, and only so-called white light sources with continuous wavelengths are available. Therefore, excitation by such X-ray irradiation has a lower excitation efficiency than light excitation, for which monochromatic light sources are widely used, and a means for increasing the amount of singlet oxygen generated is needed.

At least one aspect of the present disclosure is directed to providing a method for generating singlet oxygen that can solve the above problems and can generate a larger amount of singlet oxygen compared to conventional methods.

At least one aspect of the present disclosure is directed to providing a system for generating singlet oxygen that

can solve the above problems and can generate a larger amount of singlet oxygen compared to conventional systems.

At least one aspect of the present disclosure is directed to providing a kit for generating singlet oxygen that can solve the above problems and can generate a larger amount of singlet oxygen compared to conventional kits.

At least one aspect of the present disclosure is directed to providing an agent for generating singlet oxygen that can solve the above problems and can generate a larger amount of singlet oxygen compared to conventional agents.

## SOLUTION TO PROBLEM

According to at least one embodiment of the present disclosure, there is provided a method for generating singlet oxygen, the method including a step of generating singlet oxygen by irradiating a phthalocyanine compound crystal with electromagnetic radiation, wherein in an X-ray diffraction pattern of the phthalocyanine compound crystal obtained by X-ray diffraction measurement using CuKα rays, a peak is present within a diffraction angle 20 range of 5° to 35°, and a full width at half maximum of a peak with highest intensity within the range is not more than 2.50°.

Also, according to at least one embodiment of the present disclosure, there is provided a system for generating singlet oxygen, the system comprising: a phthalocyanine compound crystal; and a device having a radiation source for irradiating the crystal with electromagnetic radiation, wherein in an X-ray diffraction pattern of the phthalocyanine compound crystal obtained by X-ray diffraction measurement using CuKα rays, a peak is present within a diffraction angle 2θ range of 5° to 35°, and a full width at half maximum of a peak with highest intensity within the range is not more than 2.50°, and singlet oxygen is generated from the crystal.

Also, according to at least one embodiment of the present disclosure, there is provided a kit for generating singlet oxygen, the kit comprising: a phthalocyanine compound crystal; and a radiation source for irradiating the crystal with electromagnetic radiation, wherein in an X-ray diffraction pattern of the phthalocyanine compound crystal obtained by X-ray diffraction measurement using CuKα rays, a peak is present within a diffraction angle 20 range of 5° to 35°, and a full width at half maximum of a peak with highest intensity within the range is not more than 2.50°, and singlet oxygen is generated from the crystal.

Also, according to at least one embodiment of the present disclosure, there is provided an agent for generating singlet oxygen, the agent comprising a phthalocyanine compound crystal, wherein in an X-ray diffraction pattern of the phthalocyanine compound crystal obtained by X-ray diffraction measurement using CuKα rays, a peak is present within a diffraction angle 2θ range of 5° to 35°, and a full width at half maximum of a peak with highest intensity within the range is not more than 2.50°, and singlet oxygen is generated by irradiating the crystal with electromagnetic radiation.

## ADVANTAGEOUS EFFECTS OF INVENTION

According to the present disclosure, it is possible to provide a method for generating singlet oxygen, a system for generating singlet oxygen, a kit for generating singlet oxygen, and an agent for generating singlet oxygen, which can generate more singlet oxygen than conventional methods by irradiating crystals of a phthalocyanine compound with electromagnetic radiation.

## BRIEF DESCRIPTION OF THE DRAWINGS

[Fig.1] Fig. 1 is an X-ray diffraction pattern of crystals of phthalocyanine compound 1 in Example.

## DESCRIPTION OF THE EMBODIMENTS

Unless otherwise specified, descriptions of numerical ranges such as "from XX to YY" or "XX to YY" in the present disclosure include the numbers at the upper and lower limits of the range. When numerical ranges are described in stages, the upper and lower limits of each of each numerical range may be combined arbitrarily. In the present disclosure, wording such as "at least one selected from the group consisting of XX, YY and ZZ" means any of: XX; YY; ZZ; a combination of XX and YY; a combination of XX and ZZ; a combination of YY and ZZ; or a combination of XX and YY and ZZ.

The present disclosure relates to a method for generating singlet oxygen, the method including a step of generating singlet oxygen by irradiating a phthalocyanine compound crystal with electromagnetic radiation, wherein in an X-ray diffraction pattern of the phthalocyanine compound crystal obtained by X-ray diffraction measurement using CuKα rays, a peak is present within a diffraction angle 2θ range of 5° to 35°, and a full width at half maximum of a peak with the highest intensity within the range is not more than 2.50°.

According to the studies of the present inventors, the above features make it possible to generate more singlet oxygen than with a conventional phthalocyanine compound that does not have crystallinity.

The details are described below.

In order to increase the amount of singlet oxygen generated by irradiation with electromagnetic radiation, an

approach that increases the permeability of the medium for electromagnetic radiation and increases the amount of electromagnetic radiation that reaches the phthalocyanine compound can be considered. Another approach would be to improve the sensitivity of the phthalocyanine compound to electromagnetic radiation. Specifically, for light, the conjugated rings would be expanded to match the wavelength of the excitation light, and for X-rays, the mass absorption coefficient of heavy elements would be increased.

A more macroscopic and simple approach would be to simply increase the amount of phthalocyanine compound molecules present. However, there is a limit to how much the concentration of single molecules can be increased, depending on the system. In that case, the amount of colorant molecules present in the system can be dramatically increased by changing the phthalocyanine compound from a molecular to a more macroscopic particle state, in other words, by changing the colorant from a dye to a pigment.

However, where the phthalocyanine compound is changed from a single molecule to a particle state, which is simply an aggregate of these molecules, the phthalocyanine compound deep inside the particle, which cannot come into contact with oxygen, is unlikely to contribute, even if excited, to the production of singlet oxygen.

Therefore, the present inventors attempted to increase the amount of singlet oxygen generated by efficiently transmitting triplet excitons generated in phthalocyanine compound molecules deep inside, where these molecules cannot come into contact with oxygen, to phthalocyanine compound molecules near the surface where the molecules can come into contact with oxygen. As a result of their investigation, it was found that the efficiency of singlet oxygen generation can be increased by crystallizing the phthalocyanine compound molecules and suitably controlling the crystallinity thereof.

Below is a specific description of one embodiment of the present disclosure.

In the present disclosure, a phthalocyanine compound crystal is one in which, in an X-ray diffraction pattern obtained by X-ray diffraction using CuK$\alpha$ rays, a peak is present within a diffraction angle 20 range of 5° to 35°, and a full width at half maximum of a peak with the highest intensity within the range is not more than 2.50°.

The full width at half maximum of the peak with the highest intensity is preferably not more than 0.70°, and more preferably not more than 0.50°.

In the present disclosure, the smaller the full width at half maximum of the peak with the highest intensity within a diffraction angle 2$\theta$ range of 5° to 35° in the X-ray diffraction pattern, the higher the degree of crystallinity of the phthalocyanine compound crystal, and the higher the degree of crystallinity of the phthalocyanine compound crystal, the more effective the propagation of triplet excitons, which is preferable.

Furthermore, for the same reason, in the X-ray diffraction pattern using CuK$\alpha$ rays, the full widths at half maximum of the top two peaks with the highest intensity in the 20 range of 5° to 35° are preferably not more than 2.50°, more preferably not more than 0.70°, and even more preferably not more than 0.50°.

The top two peaks with the highest intensity are considered to be derived from the main diffraction planes inherent to the crystal structure, such as diffraction in the molecular axis direction perpendicular to the molecular stacking direction and diffraction in the $\pi$-stack direction. By measuring the full widths at half maximum of these two peaks, the degree of crystallinity of the phthalocyanine compound crystals can be determined more accurately.

The lower limit of the full width at half maximum is not particularly limited, and the smaller the value, the better. It is preferably at least 0.20°, more preferably at least 0.10°, and even more preferably at least 0.05°. The full width at half maximum is preferably 0.05° to 2.50°, 0.05° to 0.70°, or 0.05° to 0.50°.

It is preferable that the electromagnetic radiation have a wavelength of 0.001 nm to 10 nm because the phthalocyanine compound deep inside the particle is also easily excited. A wavelength of 0.001 nm to 2.5 nm is more preferable, and a wavelength of 0.001 nm to 1.5 nm is particularly preferable.

Specific examples of electromagnetic radiation having the above-mentioned preferred wavelength include X-rays and gamma rays.

The wavelength of the electromagnetic radiation can be measured using a semiconductor detector or the like.

The number-average particle diameter of the phthalocyanine compound crystals is preferably at least 30 nm, because the effect of crystallization is fully exhibited compared to amorphous structures when a certain number of molecules is ensured. Furthermore, since the attenuation of triplet excitons cannot be completely eliminated even if the crystallinity is increased, the overall particle diameter is preferably not more than 300 nm. More preferably, it is not more than 200 nm.

The number-average particle diameter of the phthalocyanine compound crystals can be adjusted, as appropriate, by adjusting the output and processing time when dispersing the phthalocyanine compound crystals with an ultrasonic homogenizer or the like.

The number-average particle diameter is more preferably from 30 nm to 200 nm, and even more preferably from 40 nm to 160 nm.

The number-average particle diameter of the phthalocyanine compound crystals can be measured by dynamic light scattering.

In the present disclosure, values measured using a dynamic light scattering particle size distribution meter Nanotrac UPA-EX150 (manufactured by Nikkiso Co., Ltd.) are used. Specifically, the range is set to 0.001 $\mu$m to 10 $\mu$m,

and the measurement is performed according to the following procedure.

Where the measurement sample is not dispersed in water, a dispersion liquid in which the measurement sample is dispersed is added to an aqueous solution containing a surfactant, such as Family Fresh (manufactured by Kao Corporation), to prevent aggregation, followed by stirring. After stirring, the measurement sample is poured into the above-mentioned device, and two measurements are performed to obtain the average value.

The measurement conditions are a measurement time of 30 sec, a sample particle refractive index of 1.49, water as the dispersion medium, and a dispersion medium refractive index of 1.33.

It is preferable that the phthalocyanine compound contains an atom with an atomic number of at least 11 because the sensitivity to electromagnetic radiation can be improved, and the atomic number of at least 13 is more preferable. The phthalocyanine compound may also contain multiple types of atoms with the atomic number of at least 11. The type and number of types of atoms with the atomic number of at least 11 are selected, as appropriate, from the viewpoints of the application, the electromagnetic radiation light source, the contrast with the medium, etc.

The atoms with an atomic number of from 11 to 85 are preferred, and those with an atomic number of from 13 to 85 are more preferred.

Specific examples of atoms with the atomic number of at least 11 include at least one selected from the group consisting of Ga, Al, I, and Ti.

From the viewpoint of sensitivity to electromagnetic radiation, it is preferable that the phthalocyanine compound be a metal phthalocyanine compound having a central metal atom.

The central metal atom preferably has an atomic number of at least 11, more preferably at least 13 because the sensitivity to electromagnetic radiation is increased. The type of atom with the atomic number of at least 11 is suitably selected from the viewpoints of the application, the electromagnetic radiation source, the contrast with the medium, etc.

It is more preferable that the atom has an atomic number of from 11 to 85, and even more preferable from 13 to 85.

Specific examples of atoms with the atomic number of at least 11 include at least one selected from the group consisting of Ga, Al, and Ti.

More preferably, the phthalocyanine compound crystal is a crystal of a gallium phthalocyanine compound or a titanyl phthalocyanine compound.

A metal phthalocyanine compound having an axial ligand is preferred from the viewpoint of controlling crystallinity.

From the viewpoint of controlling crystallinity and improving sensitivity to electromagnetic radiation, it is preferable that the axial ligand of the metal phthalocyanine compound be a halogen atom or a hydroxy group.

Examples of halogen atoms are fluorine, chlorine, bromine, and iodine.

It is preferable that the metal phthalocyanine compound crystal be a hydroxygallium phthalocyanine crystal, since triplet excitons propagate more effectively.

Phthalocyanine molecules are generally known to have a flat shape, and $\pi$ orbitals thereof extend in a direction perpendicular to the molecular plane (molecular axial direction).

For this reason, phthalocyanine crystals have a columnar structure in which planes are stacked to face each other due to the intermolecular interaction of the $\pi$ bonding properties of planar molecules.

Therefore, many phthalocyanine crystals, especially V-type hydroxygallium phthalocyanine crystals, have characteristic X-ray diffraction patterns.

The peak at a diffraction angle $2\theta = 7.4° \pm 0.3°$ in the X-ray diffraction pattern using CuK$\alpha$ rays is believed to be due to diffraction at the (0,1,0) plane based on the results of Rietveld crystal structure analysis, and it has been elucidated that this is a diffraction plane in the molecular axis direction.

Similarly, the peak at $28.2° \pm 0.3°$ is believed to be due to diffraction at the (2,-1,-2) plane, and it has been elucidated that this is a diffraction plane in the $\pi$ stack direction.

These peaks at diffraction angles $2\theta$ make it possible to identify the crystal structure of the phthalocyanine compound.

The elemental species of the metal atom, central metal atom, and axial ligand in the phthalocyanine compound crystal can be identified, for example, by X-ray fluorescence analysis. Specifically, the measurement device used is a wavelength dispersive X-ray fluorescence analyzer "Axios" (manufactured by PANalytical Co., Ltd.) and the accompanying dedicated software "SuperQ ver. 4.0F" (manufactured by PANalytical Co., Ltd.) for setting measurement conditions and analyzing measurement data.

Rh is used as the anode of the X-ray tube, the measurement atmosphere is vacuum, the measurement diameter (collimator mask diameter) is 27 mm, and the measurement time is 10 sec. A known detector such as a proportional counter (PC) or scintillation counter (SC) is used as the detector.

Whether the detected metal atom type is a central metal atom is determined, for example, by comparing the absorption spectrum with an existing database of metal phthalocyanines. A V-560 manufactured by JASCO Corporation or the like is used as the measurement device.

Whether the detected nonmetallic atom with an atomic number of at least 11 is an axial ligand or bonded by

replacing hydrogen on the phthalocyanine ring is determined by whether a decrease in the amount of hydrogen on the phthalocyanine ring is observed by $^{1}$H-NMR measurement.

The X-ray diffraction pattern of the phthalocyanine compound crystal detected using CuKα rays can be obtained by X-ray diffraction measurement.

Specifically, X-ray diffraction measurement is performed with the following equipment and measurement conditions.

Measuring equipment used: X-ray diffraction device RINT-TTRII, manufactured by Rigaku Electric Co., Ltd.,
X-ray tube: Cu
X-ray wavelength: 0.15418 nm
Spectral line: Kα1
Tube voltage: 50 KV
Tube current: 300 mA
Scanning method: 20 scan
Scanning speed: 4.0°/min
Sampling interval: 0.02°
Start angle 2θ: 5.0°
Stop angle 2θ: 35.0°
Goniometer: Rotor horizontal goniometer (TTR-2)
Attachment: Capillary rotating sample stage
Filter: None
Detector: Scintillation Counter
Incident monochromator: Used
Slit: Variable slit (parallel beam method)
Counter monochromator: Not used
Divergence slit: Open
Divergence vertical limiting slit: 10.00 mm
Scattering slit: Open
Receiving slit: Open

The phthalocyanine crystal preferably contains a compound represented by a formula (1) below, because the charge trapping sites are offset by the intramolecular polarity of the compound, so that the propagation of triplet excitons occurs effectively and the amount of singlet oxygen generated increases.

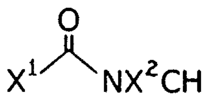 (1)

(In formula (1), $X^1$ represents a hydrogen atom, a methyl group, or an ethyl group, and $X^2$ represents a hydrogen atom, a methyl group, or an ethyl group.)

Specific examples of the compound represented by formula (1) include at least one selected from the group consisting of N-methylformamide and N,N-dimethylformamide.

Furthermore, in the compounds represented by formula (1), it is preferable that $X^2$ be a hydrogen atom. That is, the compounds represented by a formula (2) below are preferred.

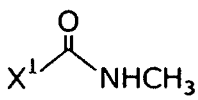 (2)

(In formula (2), $X^1$ represents a hydrogen atom, a methyl group, or an ethyl group.)

N-methylformamide is a specific example of compounds represented by formula (2).

From the viewpoint of the above effects, it is preferable that the content of the compound represented by formula (1) in the phthalocyanine crystal be from 0.10% by mass to 1.70% by mass. More preferably, this content is from 0.20% by mass to 1.20% by mass.

The content of the compound represented by formula (1) can be adjusted by the processing time in the crystal conversion step described below. The content of the compound represented by formula (1) tends to increase when the processing time is shortened.

The phthalocyanine compound crystals will be specifically described below.

In addition to phthalocyanine, one or more known phthalocyanine compounds can be used as the phthalocyanine compound without any particular restrictions.

Examples of metal phthalocyanines with coordination to elements with the atomic number of at least 11 include aluminum phthalocyanine, silicon phthalocyanine, titanyl phthalocyanine, vanadium phthalocyanine, chromium phthalocyanine, manganese phthalocyanine, iron phthalocyanine, cobalt phthalocyanine, nickel phthalocyanine, copper phthalocyanine, zinc phthalocyanine, gallium phthalocyanine, germanium phthalocyanine, zirconium phthalocyanine, niobium phthalocyanine, molybdenum phthalocyanine, rhodium phthalocyanine, palladium phthalocyanine, silver phthalocyanine, cadmium phthalocyanine, indium phthalocyanine, tin phthalocyanine, barium phthalocyanine, hafnium phthalocyanine, tungsten phthalocyanine, iridium phthalocyanine, platinum phthalocyanine, gold phthalocyanine, mercury phthalocyanine, lead phthalocyanine, europium phthalocyanine, etc.

The metal phthalocyanines may have an axial ligand. Examples of the axial ligand include a halogen atom, a hydroxy group, an optionally substituted alkoxy group, and a derivative of a compound having an alkoxy group, an optionally substituted silanolate group, and a derivative of a compound having a silanolate group.

Examples of metal phthalocyanines having a halogen atom as an axial ligand include chlorometal phthalocyanine crystals, bromometal phthalocyanine crystals, and iodometal phthalocyanine crystals. In particular, from the viewpoint of sensitivity to electromagnetic radiation, iodometal phthalocyanine crystals are preferred.

Chlorometal phthalocyanine crystals have a chlorine atom as an axial ligand. Bromometal phthalocyanine crystals have a bromine atom as an axial ligand. Iodophthalocyanine crystals have an iodine atom as an axial ligand.

A hydroxymetal phthalocyanine having a hydroxy group as an axial ligand can be obtained by, for example, dissolving a halogenated phthalocyanine in concentrated sulfuric acid to liberate the halogen, and then treating it with aqueous ammonia.

These metal phthalocyanine compounds having halogen atoms as axial ligands can be obtained through a treatment step of mixing hydroxy metal phthalocyanine with an aqueous solution of hydrochloric acid, bromic acid, iodic acid, or the like having the halogen atoms.

From the viewpoint of reactivity, the concentration of the aqueous solution of hydrochloric acid, bromic acid, iodic acid, or the like mixed with the hydroxy metal phthalocyanine in this treatment step is preferably at least 10% by mass, and more preferably at least 30% by mass. The step of mixing the aqueous solution of hydrochloric acid, bromic acid, iodic acid, or the like with the hydroxy metal phthalocyanine can include a milling process or a stirring process.

In addition, the phthalocyanine compound will no longer have an axial ligand if treated with a strong acid such as concentrated sulfuric acid.

Substituted phthalocyanines in which part of the phthalocyanine ring of phthalocyanine and metal phthalocyanine is substituted can also be used. Specific examples of the substituent include at least one selected from the group consisting of halogens such as chlorine, bromine, and iodine, hydroxyl, amino, imino, cyano, nitro, sulfo, optionally substituted alkyl, alkenyl, alkynyl, aryl, arylalkyl, heteroaryl, alkoxy, alkenyloxy, alkynyloxy, aryloxy, and heteroaryloxy, acyl, acetyl, formyl, carboxy, and carbonyl.

The phthalocyanine compound crystals can be obtained in a crystal conversion step in which a conversion solvent, for example, a compound represented by formula (1), is added to the phthalocyanine compound and milling is performed to convert the phthalocyanine compound crystals. By milling using a conversion solvent containing the compound represented by formula (1) as the conversion solvent, the compound represented by formula (1) can be included in the phthalocyanine compound crystals.

The milling treatment performed herein is carried out using a milling device such as a sand mill or a ball mill together with a dispersing agent such as glass beads, steel beads, or alumina balls. The amount of the compound represented by formula (1) to be added in the milling treatment is preferably 5 times to 30 times the amount of the phthalocyanine compound on a mass basis.

In addition to the compound represented by formula (1) above, for example, an amide solvent such as N,N-dimethylformamide, N,N-dimethylacetamide, and 1-methyl-2-pyrrolidone, a halogen solvent such as chloroform, an ether solvent such as tetrahydrofuran, or a sulfoxide solvent such as dimethyl sulfoxide may be used as the conversion solvent. The amount of the solvent used is preferably 5 times to 30 times the amount of the phthalocyanine compound on a mass basis.

If too much conversion solvent is used, the concentration becomes low, which reduces the processing efficiency during milling and tends to make it difficult to increase the degree of crystallinity of the phthalocyanine compound. Conversely, if the amount of conversion solvent used is too small and the concentration is too high, the processing efficiency will be too high, and the physical impact will be too strong, which is likely to destroy the crystals themselves and to reduce the degree of crystallinity.

The milling time is preferably at least 4 h, more preferably at least 200 h, even more preferably from 200 h to 2000 h. By suitably selecting the type of conversion solvent, the amount used, as well as the milling time, the degree of crystallinity of the phthalocyanine compound can be adjusted.

<Method for Quantification of Compound Represented by Formula (1), NMR>

Whether the phthalocyanine compound crystal contains the conversion solvent, particularly the compound represented by formula (1), inside the crystal is determined by subjecting the obtained phthalocyanine compound crystal to $^1$H-NMR measurement and analyzing the data.

Specifically, the presence or absence of the compound represented by formula (1) and the content thereof are quantified from the presence or absence of a peak derived from the compound, among the measured peaks of the phthalocyanine compound, and the area thereof.

NMR measurement of the crystal of phthalocyanine compound is performed under the following conditions.

[$^1$H-NMR Measurement]

Measuring device used: product name: AVANCEIII 500, manufactured by BRUKER Corp.
Solvent: Bisulfuric acid ($D_2SO_4$)
Number of integrations: 2000

<Method for Generating Singlet Oxygen>

A method for generating singlet oxygen by irradiating the phthalocyanine compound crystals according to the present disclosure with electromagnetic radiation can be exemplified by, but is not limited to, a procedure including the following steps (I) and (II).

Step (I): A step of placing the phthalocyanine compound crystals in an atmosphere in which oxygen is present.

Step (II): A step of irradiating the phthalocyanine compound crystals placed in step (I) with electromagnetic radiation.

In step (I), the method for placing the crystals in an atmosphere in which oxygen is present is not particularly limited as long as the phthalocyanine compound crystals come into contact with oxygen. For example, there are a method of allowing the phthalocyanine compound crystals to stand in an air atmosphere, a method of coating the crystals for dispersion in water (described hereinbelow), dispersing the crystals in water, and then mixing the crystals with a liquid containing dissolved oxygen gas. It is preferable to disperse the phthalocyanine compound crystals in a liquid containing oxygen-containing water.

When the phthalocyanine compound crystals are dispersed in a liquid containing water, the concentration is not particularly limited, and may be 0.0001% by mass to 50% by mass, or 0.001% by mass to 20% by mass.

In step (II), the method for irradiating with electromagnetic radiation is not particularly limited as long as electromagnetic radiation of sufficient intensity is radiated to generate singlet oxygen. Specifically, from the viewpoint of suppressing attenuation and scattering of electromagnetic radiation, it is most preferable to use an electromagnetic radiation irradiating device and perform the process in a vacuum where there is theoretically no substance between the phthalocyanine compound crystals and the irradiating device, but in practice it is preferable to perform the process in the atmosphere, water, or other medium under conditions where there is as little electromagnetic radiation absorber between the irradiating device and the crystals as possible.

The accumulated light quantity of electromagnetic radiation is preferably 0.01 Gy to 1000 Gy, and more preferably 0.1 Gy to 100 Gy.

The irradiation time is not particularly limited, and is set, as appropriate, depending on the equipment used and the amount of singlet oxygen to be generated.

The distance between the phthalocyanine compound crystals and the source of electromagnetic radiation is not particularly limited, but is set, as appropriate, depending on the degree of absorption and scattering of electromagnetic radiation by the substance present between the irradiating device and the phthalocyanine compound crystals.

<Coating Method for Dispersion in Water>

The phthalocyanine compound crystal may have a coating layer of a compound having a suitable function on the surface depending on the application. The compound on the surface may be chemically or electrostatically bonded to the phthalocyanine compound crystal surface. It may also be introduced as a substituent of the phthalocyanine ring. It may also be introduced as an axial ligand of the phthalocyanine compound molecule on the surface. Alternatively, it may be encapsulated in a micelle of an amphiphilic molecule.

For example, in applications requiring dispersibility in water or hydrophilicity, the surface of the phthalocyanine compound crystal may be coated with a compound having at least one of the sites having hydrogen bonds or the ability to

form a hydration layer, or these sites may be substituted or introduced into the phthalocyanine compound. Specific examples of the coating include compounds having a hydroxy group, an amide bond, ethylene oxide, a betaine structure, and a phosphoric acid ester.

As a specific embodiment of the coating, for example, it is possible to coat the phthalocyanine compound crystal surface with a monomer containing glycidyl methacrylate, carry out polymerization, and then subject a monomer having a hydroxy group, such as 3-mercapto-1,2-propanediol, to ring-opening polymerization with an epoxy ring derived from glycidyl methacrylate to introduce a hydroxy group onto the surface.

A hydroxy group can also be introduced into the phthalocyanine ring of the phthalocyanine compound on the crystal surface. A hydrophilic ligand such as {3-[bis(3-sulfopropyl)(3-sulfonatopropyl)azaniumyl]propylldimethylsilanolate can also be introduced as an axial ligand of the phthalocyanine compound on the crystal surface.

In applications requiring dispersibility in water, the phthalocyanine compound can be coated with a compound having sites that generate electrostatic repulsion, or these sites can be substituted or introduced into the phthalocyanine compound. Examples of the sites that produce electrostatic repulsion include carboxyl group, sulfonic acid group, amino group, and imino group.

Encapsulation in a micelle such as a surfactant is also possible. Specific examples of surfactants include cetyltrimethylammonium chloride, tetramethylammonium chloride, tetrabutylammonium chloride, dodecyldimethylbenzylammonium chloride, alkyltrimethylammonium chloride, octyltrimethylammonium chloride, hexadecyltrimethylammonium chloride, cetyltrimethylammonium bromide, alkyltrimethylammonium bromide, hexadecyltrimethylammonium bromide, benzalkonium bromide, sodium dodecyl sulfate, sodium lauryl sulfate, tetraethylene glycol monododecyl ether, pentaethylene glycol monododecyl ether, octaethylene glycol monododecyl ether, Newcol (registered trademark) (manufactured by Nippon Nyukazai Co., Ltd.), and Emulgen (registered trademark) (manufactured by Kao Corporation).

In applications requiring biocompatibility, the surface of the phthalocyanine compound crystal may be coated with at least one molecule selected from the group consisting of hydrophilic polymers such as polyethylene glycol, polyvinylpyrrolidine, polyvinyl methyl ether, polymethyloxazoline, polyethyloxazoline, polyhydroxy-propyloxazoline, polyhydroxypropyl methacrylamide, polymethacrylamide, polydimethylacrylamide, polyhydroxypropyl methacrylate, polyhydroxy-ethyl acrylate, hydroxymethyl cellulose, hydroxyethyl cellulose, and polyethylene-imine, which are covalently or electrostatically bonded, and lipids such as 1,2-dioleoyl-3-trimethylammonium propane (DOTAP), 1,2-dioleoyl-sn-glycero-3-phosphocholine (DOPC), and 1,2-distearoyl-sn-glycero-3-phosphoethanolamine-N-[amino(polyethylene glycol)] (DSPE-PEG). Other known biocompatible molecules may also be used. Encapsulation in micelles of these molecules is also possible.

<Quantification of Singlet Oxygen>

The amount of singlet oxygen generated when the phthalocyanine compound crystals are irradiated with electromagnetic radiation is quantified using an aqueous solution of 4-nitroso-N,N-dimethylaniline.

When singlet oxygen is generated by irradiating the phthalocyanine compound crystals dispersed in an aqueous solution of 4-nitroso-N,N-dimethylaniline containing histidine with electromagnetic radiation, the singlet oxygen reacts with 4-nitroso-N,N-dimethylaniline, causing a decrease in the absorbance at 440 nm in the UV-visible spectrum originating from 4-nitroso-N,N-dimethylaniline. This change in absorbance correlates with the amount of singlet oxygen generated, making quantitative measurement and comparison possible. The equipment and measurement conditions used are as follows:

Measurement solution: 4-nitroso-N,N-dimethylaniline 25 $\mu$M, histidine 10 mM
Measuring instrument used: UV-Visible spectrophotometer JASCO V-570, manufactured by JASCO Corporation
Photometric mode: Abs absorbance measurement
Response: fast
Bandwidth: 2.0 nm
Scanning speed: 200 nm/min
Data capture interval: 0.5 nm

<System and Kit for Generating Singlet Oxygen>

The above-described method for generating singlet oxygen can be implemented using a system for generating singlet oxygen comprising a phthalocyanine compound crystal and an apparatus having a radiation source for irradiating the crystal with electromagnetic radiation.

Also, a kit for generating singlet oxygen generating may be provided in which a phthalocyanine compound crystal is combined with a component such as a radiation source for irradiating the crystal with electromagnetic radiation.

Preferable embodiments of the phthalocyanine compound crystal are the same as those relating to the above-described method for generating singlet oxygen.

The apparatus having a radiation source for irradiating with electromagnetic radiation is not particularly limited as long as step (II) in the above-mentioned method for generating singlet oxygen can be implemented. For example, a system or kit can be formed by placing a phthalocyanine compound crystal at the irradiation target of a radiation source such as an X-ray tube, a linac, or other accelerator-derived radiation irradiation device, or an apparatus having a radioisotope etc.

It is preferable that there be a vacuum between the radiation source and the phthalocyanine compound crystal, but air, water, or other medium may be present, and it is preferable that there be at least as little electromagnetic radiation absorber, such as lead, as possible.

<Agent for Generating Singlet Oxygen>

A phthalocyanine compound crystal can be used as an agent for generating singlet oxygen.

Preferable embodiments of the phthalocyanine compound crystal are the same as those related to the above-mentioned method for generating singlet oxygen.

A method for formulating the phthalocyanine compound crystal as an agent for generating singlet oxygen is not particularly limited as long as the phthalocyanine compound crystal is contained in the agent for generating singlet oxygen.

The content of the phthalocyanine compound crystal in the agent for generating singlet oxygen is preferably 0.0001% by mass to 50% by mass, more preferably 0.001% by mass to 20% by mass.

The agent for generating singlet oxygen may be solid or liquid.

The agent for generating singlet oxygen may contain, for example, an excipient, a lubricant, a binder, and a disintegrant in a solid formulation, or a solvent, a solubilizer, a suspending agent, an isotonicity agent, a buffer, etc. in a liquid formulation. Furthermore, if necessary, additives such as preservatives, antioxidants, colorants, adsorbents, and wetting agents may be contained, as appropriate, in appropriate amounts.

The phthalocyanine compound crystals in the agent for generating singlet oxygen may be dispersed in a medium such as water in the form of nanoparticles, or may be fixed to the surface of a carrier. The carrier may be a bulk solid phase, or may be particles such as other nanoscale particles, microscale particles, or liposomes. The phthalocyanine compound crystals may also be physically enclosed in a gel-shaped polymer such as hyaluronic acid.

The surface of the phthalocyanine compound crystal may have a coating layer for the purpose of improving the medium dispersibility and stability, or the crystals may be encapsulated in micelles. In addition, molecules of different functionality, such as molecules that correspond to ligands with targeting functions in vivo, may be bonded or added.

## EXAMPLES

The present disclosure will be described in more detail below with specific examples. However, the present disclosure is not limited to these. The "parts" shown below mean "parts by mass."

<Method for Producing Crystals of Phthalocyanine Compound 1>

In a nitrogen flow atmosphere, 5.46 parts of orthophthalonitrile and 45 parts of $\alpha$-chloronaphthalene were put into a reaction vessel, which was then heated to a temperature of 30°C and maintained at this temperature. Next, 3.75 parts of gallium trichloride was put in at this temperature (30°C). The concentration of water in the mixed liquid at the time of the addition was 150 ppm. The temperature was then raised to 200°C. Next, the components were reacted at 200°C for 4.5 h under a nitrogen flow atmosphere, followed by cooling. The product was filtered when the temperature reached 150°C. The resulting filtrate was dispersed and washed using N,N-dimethylformamide at a temperature of 140°C for 2 h and then filtered. The resulting filtrate was washed with methanol and then dried to obtain chlorogallium phthalocyanine.

A total of 4.65 parts of the obtained chlorogallium phthalocyanine was dissolved in 139.5 parts of concentrated sulfuric acid at a temperature of 10°C, and the solution was dropped into 620 parts of ice water while stirring to cause reprecipitation, and then filtered under reduced pressure using a filter press. At this time, No. 5C (manufactured by Advantec Co., Ltd.) was used as the filter. The obtained wet cake (filtrate) was dispersed and washed with 2% ammonia water for 30 min and then filtered using a filter press. Next, the obtained wet cake (filtrate) was dispersed and washed with ion-exchanged water and then filtered three times using a filter press. Finally, freeze-drying was performed to obtain hydroxygallium phthalocyanine.

A total of 0.5 parts of the obtained hydroxygallium phthalocyanine and 9.5 parts of N-methylformamide (product code: F0059, manufactured by Tokyo Chemical Industry Co., Ltd.), which is a compound represented by formula (1), as a conversion solvent, together with 15 parts of glass beads having a diameter of 0.8 mm, were milled in a ball mill at room temperature (23°C) for 600 h. At this time, a standard bottle (product code: PS-6, manufactured by Kashiwa Glass Co.,

Ltd.) was used as the container, and the milling was performed under the condition of the container rotating at 60 revolutions per minute. Gallium phthalocyanine crystals were taken out from this dispersion liquid using tetrahydrofuran and filtered, and the material on the filter was thoroughly washed with tetrahydrofuran. The filtered product was vacuum dried to obtain 0.45 parts of crystals of phthalocyanine compound 1.

The X-ray diffraction pattern of the obtained crystals is shown in Fig. 1. It can be seen that the crystals have peaks at diffraction angles $2\theta = 7.4° \pm 0.2°$ and $28.3° \pm 0.2°$ in CuK$\alpha$ X-ray diffraction. The full widths at half maximum of the top two peaks with high intensity in the CuK$\alpha$ X-ray diffraction pattern are shown in Table 1.

When the crystals were dissolved in heavy sulfuric acid and $^1$H-NMR measurement was performed, a peak due to N-methylformamide was observed in addition to the peak due to the molecules of phthalocyanine compound 1. Since N-methylformamide is a liquid at room temperature and is compatible with tetrahydrofuran, it can be understood that N-methylformamide is contained in the crystals of phthalocyanine compound 1. The content of N-methylformamide in the crystals of phthalocyanine compound 1 was 0.9% by mass calculated from the proton ratio.

The presence or absence of metal atoms with the atomic number of at least 11, the type of central metal atom, and the type of axial ligand of the obtained crystals were comprehensively determined by fluorescent X-ray analysis, absorption spectrum analysis, and $^1$H-NMR measurement. The results are shown in Table 1.

<Method for Producing Crystals of Phthalocyanine Compound 2>

Crystals of phthalocyanine compound 2 were obtained in the same manner as in the method for producing crystals of phthalocyanine compound 1, except that the milling treatment time was changed to 200 h. Table 1 shows the full widths at half maximum of the top two peaks with high intensity in the X-ray diffraction pattern of CuK$\alpha$ rays, the type and content of the compound represented by formula (1), the presence or absence of metal atoms with the atomic number of at least 11, the type of central metal atom, and the type of axial ligand in the obtained crystals of phthalocyanine compound 2.

<Method for Producing Crystals of Phthalocyanine Compound 3>

Crystals of phthalocyanine compound 3 were obtained in the same manner as in the method for producing crystals of phthalocyanine compound 1, except that the milling treatment was changed to 1000 h. Table 1 shows the full width at half maximum of the top two peaks with high intensity in the X-ray diffraction pattern of CuK$\alpha$ rays, the type and content of the compound represented by formula (1), the presence or absence of metal atoms with the atomic number of at least 11, the type of central metal atom, and the type of axial ligand in the obtained crystals of phthalocyanine compound 3.

<Method for Producing Crystals of Phthalocyanine Compound 4>

Crystals of phthalocyanine compound 4 were obtained in the same manner as in the method for producing crystals of phthalocyanine compound 1, except that the conversion solvent N-methylformamide was changed to N,N-dimethylformamide (product code: D0722, manufactured by Tokyo Chemical Industry Co., Ltd.). Table 1 shows the full widths at half maximum of the top two peaks with high intensity in the X-ray diffraction pattern of CuK$\alpha$ rays, the type and content of the compound represented by formula (1), the presence or absence of metal atoms with the atomic number of at least 11, the type of central metal atom, and the type of axial ligand in the obtained crystals of phthalocyanine compound 4.

<Method for Producing Crystals of Phthalocyanine Compound 5>

A total of 10 parts of crystals of phthalocyanine compound 1 and 200 parts of iodic acid with a concentration of 35% by mass and a temperature of 23°C were mixed and stirred with a magnetic stirrer for 90 min. The amount of iodic acid mixed was 118 mol of hydrogen iodide per 11 mol of the phthalocyanine compound. After stirring, the mixture was dropped into 1000 parts of ion-exchanged water cooled with ice water and stirred with a magnetic stirrer for 30 min. This was filtered under reduced pressure. No. 5C (manufactured by Advantec Co., Ltd.) was used as the filter. Then, dispersion and washing were performed four times with ion-exchanged water at a temperature of 23°C. In this way, 9 parts of crystals of phthalocyanine compound 5 were obtained. Table 1 shows the full widths at half maximum of the top two peaks with high intensity in the X-ray diffraction pattern of CuK$\alpha$ rays, the type and content of the compound represented by formula (1), the presence or absence of metal atoms with the atomic number of at least 11, the type of central metal atom, and the type of axial ligand in the obtained crystals of phthalocyanine compound 5.

<Method for Producing Crystals of Phthalocyanine Compound 6>

Crystals of phthalocyanine compound 6 were obtained in the same manner as in the method for producing crystals of phthalocyanine compound 4, except that 3.75 parts of gallium trichloride was changed to 2.84 parts of aluminum

chloride. Table 1 shows the full widths at half maximum of the top two peaks with high intensity in the X-ray diffraction pattern of CuKα rays, the type and content of the compound represented by formula (1), the presence or absence of metal atoms with the atomic number of at least 11, the type of central metal atom, and the type of axial ligand in the obtained crystals of phthalocyanine compound 6.

<Method for Producing Crystals of Phthalocyanine Compound 7>

Crystals of phthalocyanine compound 7 were obtained in the same manner as in the method for producing crystals of phthalocyanine compound 4, except that the conversion solvent N,N-dimethylformamide was changed to tetrahydrofuran. Table 1 shows the full widths at half maximum of the top two peaks with high intensity in the X-ray diffraction pattern of CuKα rays, the type and content of the compound represented by formula (1), the presence or absence of metal atoms with the atomic number of at least 11, the type of central metal atom, and the type of axial ligand in the obtained crystals of phthalocyanine compound 7.

<Method for Producing Crystals of Phthalocyanine Compound 8>

Crystals of phthalocyanine compound 8 were obtained in the same manner as in the method for producing crystals of phthalocyanine compound 7, except that the milling treatment time was changed to 300 h. Table 1 shows the full widths at half maximum of the top two peaks with high intensity in the X-ray diffraction pattern of CuKα rays, the type and content of the compound represented by formula (1), the presence or absence of metal atoms with the atomic number of at least 11, the type of central metal atom, and the type of axial ligand in the obtained crystals of phthalocyanine compound 8.

<Method for Producing Crystals of Phthalocyanine Compound 9>

In a nitrogen flow atmosphere, 5.46 parts of orthophthalonitrile and 45 parts of α-chloronaphthalene were put into a reaction vessel, which was then heated to a temperature of 30°C and maintained at this temperature. Next, 3.75 parts of copper dichloride was put in at this temperature (30°C). The concentration of water in the mixed liquid at the time of the addition was 150 ppm. The temperature was then raised to 200°C. Next, the components were reacted at 200°C for 4.5 h under a nitrogen flow atmosphere, followed by cooling. The product was filtered when the temperature reached 150°C. The resulting filtrate was dispersed and washed using N,N-dimethylformamide at a temperature of 140°C for 2 h, and then filtered. The resulting filtrate was washed with methanol and then dried to obtain copper phthalocyanine.

A total of 4.65 parts of the obtained copper phthalocyanine was dissolved in 139.5 parts of concentrated sulfuric acid at a temperature of 10°C, and the solution was dropped into 620 parts of ice water while stirring to cause reprecipitation and then filtered under reduced pressure using a filter press. At this time, No. 5C (manufactured by Advantec Co., Ltd.) was used as the filter. The obtained wet cake (filtrate) was dispersed and washed with ion-exchanged water for 30 min and then filtered using a filter press. Next, the obtained wet cake (filtrate) was dispersed and washed with ion-exchanged water and then filtered three times using a filter press. Finally, freeze-drying was performed to obtain crystals of phthalocyanine compound 9. Table 1 shows the full widths at half maximum of the top two peaks with high intensity in the X-ray diffraction pattern of CuKα rays, the type and content of the compound represented by formula (1), the presence or absence of metal atoms with the atomic number of at least 11, the type of central metal atom, and the type of axial ligand in the obtained crystals of phthalocyanine compound 9.

<Method for Producing Crystals of Phthalocyanine Compound 10>

A total of 100 parts of α-chloronaphthalene, 5.0 parts of o-phthalodinitrile, and 2.0 parts of titanium tetrachloride were heated and stirred at 200°C for 3 h and then cooled to 50°C. The precipitated crystals were filtered off to obtain a paste of dichlorotitanium phthalocyanine. This was then washed under stirring in N,N-dimethylformamide heated to 100°C, and then washed twice with methanol at 60°C and filtered off. The resulting paste was further stirred in deionized water at 80°C for 1 h and filtered off to obtain 4.3 parts of titanyl phthalocyanine.

A total of 0.5 parts of the obtained titanyl phthalocyanine, 10 parts of tetrahydrofuran, and 15 parts of glass beads with a diameter of 0.9 mm were milled for 3 hours at room temperature (23°C) using a paint shaker (manufactured by Toyo Seiki Seisakusho, Ltd.) (first stage). A standard bottle (product name: PS-6, manufactured by Kashiwa Glass Co., Ltd.) was used as the container. The milled liquid was filtered with a filter (product number: N-NO. 125T, pore size: 133 μm, manufactured by NBC Meshtec Inc.) to remove the glass beads. This liquid was milled in a ball mill at room temperature (23°C) for 300 h (second stage). A standard bottle (product name: PS-6, manufactured by Kashiwa Glass Co., Ltd.) was used as the container, and the container was rotated at 120 revolutions per minute. A total of 30 parts of tetrahydrofuran was added to the treated liquid, which was then filtered and washed. The washed filtered material was then vacuum dried to obtain 0.44 parts of crystals of phthalocyanine compound 10.

Table 1 shows the full widths at half maximum of the top two peaks with high intensity in the X-ray diffraction pattern of CuKα rays, the type and content of the compound represented by formula (1), the presence or absence of metal atoms with the atomic number of at least 11, the type of central metal atom, and the type of axial ligand in the obtained crystals of phthalocyanine compound 10.

<Method for Producing Crystals of Phthalocyanine Compound 11>

In a nitrogen flow atmosphere, 5.46 parts of orthophthalonitrile and 45 parts of amyl alcohol were put into a reaction vessel, and then 7.23 parts of 1,8-diazabicyclo[5,4,0]undecene-7 was added using a dropping funnel, followed by heating and refluxing for 5 h. The temperature was then raised to 200°C. Next, the components were reacted at 200°C for 4.5 hours under a nitrogen flow atmosphere, followed by cooling. The product was filtered when the temperature reached 150°C. The resulting filtrate was dispersed and washed using N,N-dimethylformamide at 140°C for 2 h, and then filtered. The resulting filtrate was washed with methanol and dried to obtain crystals of phthalocyanine compound 11. Table 1 shows the full widths at half maximum of the top two peaks with high intensity in the X-ray diffraction pattern of CuKα rays, the type and content of the compound represented by formula (1), the presence or absence of metal atoms with the atomic number of at least 11, the type of central metal atom, and the type of axial ligand in the obtained crystals of phthalocyanine compound 11.

<Method for Producing Crystals of Phthalocyanine Compound 12>

A total of 4.65 parts of crystals of phthalocyanine compound 11 was dissolved in 139.5 parts of concentrated sulfuric acid at a temperature of 10°C, and the solution was dropped into 620 parts of ice water while stirring to cause reprecipitation and then filtered under reduced pressure using a filter press. At this time, No. 5C (manufactured by Advantec Co., Ltd.) was used as the filter. The obtained wet cake (filtrate) was dispersed and washed with 2% ammonia water for 30 min and then filtered using a filter press. Next, the obtained wet cake (filtrate) was dispersed and washed with ion-exchanged water and then filtered three times using a filter press. Finally, freeze-drying was performed to obtain crystals of phthalocyanine compound 12. Table 1 shows the full widths at half maximum of the top two peaks with high intensity in the X-ray diffraction pattern of CuKα rays, the type and content of the compound represented by formula (1), the presence or absence of metal atoms with the atomic number of at least 11, the type of central metal atom, and the type of axial ligand in the obtained crystals of phthalocyanine compound 12.

Table 1

| | Atom with atomic number of at least 11 | | Axial ligand | Full width at half maximum of peak with first highest peak intensity at diffraction angle 2θ in X-ray diffraction with CuKα rays (°) | Full width at half maximum of peak with second highest peak intensity at diffraction angle 2θ in X-ray diffraction with CuKα rays (°) | Type of compound represented by formula (1) | Content of compound represented by formula (1) (% by mass) |
|---|---|---|---|---|---|---|---|
| | Central metal atom | Other atoms | | | | | |
| Crystals of phthalocyanine compound 1 | Ga (atomic number 31) | None | Hydroxy group | 0.43 | 0.41 | N-Methylformamide | 0.90% |
| Crystals of phthalocyanine compound 2 | Ga (atomic number 31) | None | Hydroxy group | 0.44 | 0.46 | N-Methylformamide | 1.70% |

(continued)

| | Atom with atomic number of at least 11 | | Axial ligand | Full width at half maximum of peak with first highest peak intensity at diffraction angle 2θ in X-ray diffraction with CuKα rays (°) | Full width at half maximum of peak with second highest peak intensity at diffraction angle 2θ in X-ray diffraction with CuKα rays (°) | Type of compound represented by formula (1) | Content of compound represented by formula (1) (% by mass) |
|---|---|---|---|---|---|---|---|
| | Central metal atom | Other atoms | | | | | |
| Crystals of phthalocyanine compound 3 | Ga (atomic number 31) | None | Hydroxy group | 0.41 | 0.39 | N-Methylformamide | 0.10% |
| Crystals of phthalocyanine compound 4 | Ga (atomic number 31) | None | Hydroxy group | 0.48 | 0.45 | N,N-Dimethylformamide | 0.52% |
| Crystals of phthalocyanine compound 5 | Ga (atomic number 31) | I (atomic number 53) | I | 0.42 | 0.49 | N-Methylformamide | 0.81% |
| Crystals of phthalocyanine compound 6 | Al (atomic number 13) | None | Hydroxy group | 0.53 | 0.21 | N,N-Dimethylformamide | 0.39% |
| Crystals of phthalocyanine compound 7 | Ga (atomic number 31) | None | Hydroxy group | 0.65 | 0.56 | None | 0.00% |
| Crystals of phthalocyanine compound 8 | Ga (atomic number 31) | None | Hydroxy group | 0.82 | 0.74 | None | 0.00% |
| Crystals of phthalocyanine compound 9 | Cu (atomic number 29) | None | None | 2.18 | 1.36 | None | 0.00% |
| Crystals of phthalocyanine compound 10 | Ti (atomic number 22) | None | 0 | 1.04 | 1.1 | None | 0.00% |
| Crystals of phthalocyanine compound 11 | None | None | None | 1.21 | 1.39 | None | 0.00% |
| Phthalocyanine compound 12 | None | None | None | 13.8 | 4.9 | None | 0.00% |

<Example 1>

The obtained crystals of phthalocyanine compound 1 were dispersed in a 0.1 mg/mL aqueous solution of surfactant (Emulgen, manufactured by Kao Corporation) to a concentration of 1 mg/mL, and treated with an ultrasonic homogenizer (VCX-750, manufactured by Sonics & Materials Co., Ltd.) at an output of 100 kW for 30 sec to obtain a dispersion liquid of crystals of phthalocyanine compound 1. The number-average particle diameter of the crystals of phthalocyanine compound 1 was 149.2 nm.

[Evaluation of Enhancement Effect of Amount of Singlet Oxygen Generated]

The absorbance at 440 nm in the UV-visible spectrum of an aqueous solution of 25 μM 4-nitroso-N,N-dimethylaniline and 10 mM histidine was measured using a spectrophotometer. Next, this aqueous solution was irradiated for 10 sec with mixed X-rays (white X-rays) containing electromagnetic radiation with wavelengths of from 0.001 nm to 10 nm, which were emitted when a tube voltage of 60 kV and 30 mA was applied to an X-ray tube with a tungsten target anode and a beryllium transmission window, and the absorbance at 440 nm in the UV-visible spectrum was measured in the same manner. The decrease rate Rr of the absorbance at 440 nm was calculated from the UV-visible spectrum before and after irradiation.

Next, 5 mL of the dispersion liquid of the crystals of phthalocyanine compound 1 was mixed with 5 mL of an aqueous solution of 50 μM 4-nitroso-N,N-dimethylaniline and 20 mM histidine, and the absorbance at 440 nm in the UV-visible spectrum was measured using a spectrophotometer. Next, this liquid mixture was irradiated with the same white X-rays for 10 sec, the absorbance at 440 nm in the UV-visible spectrum was measured in the same manner, and the decrease rate Rx of the absorbance at 440 nm in the UV-visible spectrum before and after irradiation was calculated.

Furthermore, the enhancement effect Ege of the amount of singlet oxygen generated by the crystals of phthalocyanine compound 1 was calculated from a formula below.

$$Ege = (Rx - Rr)/Rr$$

The obtained Ege is shown in Table 2.

<Examples 2-14, Comparative Example 1>

The enhancement effect of the amount of singlet oxygen generated was evaluated in the same manner, except that the crystals of phthalocyanine compound 1 in Example 1 were replaced with crystals of phthalocyanine compounds 2 to 11 and phthalocyanine compound 12, as shown in Table 2, and the output and processing time of the ultrasonic homogenizer were changed as shown in Table 2. The number-average particle diameter of the phthalocyanine compounds and the enhancement effect Ege of the amount of singlet oxygen generated are shown in Table 2.

[Table 2]

Table 2

| | Phthalocyanine compound | Treatment with ultrasonic homogenizer | Number-average particle diameter [nm] | Enhancement effect of amount of singlet oxygen generated Ege |
|---|---|---|---|---|
| Example 1 | Crystals of Pc compound 1 | Output 100 kw/30 sec treatment | 149.2 | 42.5% |
| Example 2 | Crystals of Pc compound 2 | Output 100 kw/30 sec treatment | 151.6 | 43.8% |
| Example 3 | Crystals of Pc compound 3 | Output 100 kw/30 sec treatment | 156.2 | 40.1% |
| Example 4 | Crystals of Pc compound 4 | Output 100 kw/30 sec treatment | 147.2 | 38.2% |

(continued)

| | Phthalocyanine compound | Treatment with ultrasonic homogenizer | Number-average particle diameter [nm] | Enhancement effect of amount of singlet oxygen generated Ege |
|---|---|---|---|---|
| Example 5 | Crystals of Pc compound 5 | Output 100 kw/30 sec treatment | 153.4 | 56.0% |
| Example 6 | Crystals of Pc compound 6 | Output 100 kw/30 sec treatment | 156.9 | 30.6% |
| Example 7 | Crystals of Pc compound 7 | Output 100 kw/30 sec treatment | 151.2 | 37.0% |
| Example 8 | Crystals of Pc compound 8 | Output 100 kw/30 sec treatment | 154.7 | 35.9% |
| Example 9 | Crystals of Pc compound 9 | Output 100 kw/30 sec treatment | 158.2 | 32.2% |
| Example 10 | Crystals of Pc compound 10 | Output 100 kw/30 sec treatment | 160.4 | 37.9% |
| Example 11 | Crystals of Pc compound 11 | Output 100 kw/30 sec treatment | 157.2 | 18.7% |
| Example 12 | Crystals of Pc compound 11 | Output 100 kw/40 sec treatment | 87.9 | 23.8% |
| Example 13 | Crystals of Pc compound 11 | Output 100 kw/15 sec treatment | 283.2 | 12.7% |
| Example 14 | Crystals of Pc compound 11 | Output 100 kw/7.5 sec treatment | 354.6 | 8.2% |
| Comparative Example 1 | Pc compound 12 | Output 100 kw/30 sec treatment | 151.6 | 7.6% |

In Table 2, Pc compound refers to a phthalocyanine compound.

The present disclosure is not limited to the above embodiments, and various changes and modifications are possible without departing from the spirit and scope of the present disclosure. Therefore, the following claims are appended to make the scope of the present disclosure public.

This application claims the benefit of Japanese Patent Application No. 2023-071394, filed April 25, 2023, and Japanese Patent Application No. 2024-062199, filed April 8, 2024, which are hereby incorporated by reference herein in their entirety.

## Claims

**1.** A method for generating singlet oxygen,

the method including a step of generating singlet oxygen by irradiating a phthalocyanine compound crystal with electromagnetic radiation, wherein
in an X-ray diffraction pattern of the phthalocyanine compound crystal obtained by X-ray diffraction measurement using CuK$\alpha$ rays, a peak is present within a diffraction angle 2$\theta$ range of 5° to 35°, and
a full width at half maximum of a peak with highest intensity within the range is not more than 2.50°.

**2.** The method for generating singlet oxygen according to claim 1, wherein a wavelength of the electromagnetic radiation is 0.001 to 10 nm.

**3.** The method for generating singlet oxygen according to claim 1 or 2, wherein a number-average particle diameter of the crystals is 30 to 300 nm.

**4.** The method for generating singlet oxygen according to any one of claims 1 to 3, wherein the phthalocyanine compound contains an atom with an atomic number of at least 11.

**5.** The method for generating singlet oxygen according to any one of claims 1 to 4, wherein the phthalocyanine compound is a metal phthalocyanine compound having a central metal atom.

**6.** The method for generating singlet oxygen according to claim 5, wherein the central metal atom is an atom with an atomic number of at least 11.

**7.** The method for generating singlet oxygen according to claim 5 or 6, wherein the metal phthalocyanine compound has an axial ligand.

**8.** The method for generating singlet oxygen according to claim 7, wherein the axial ligand is a halogen atom or a hydroxy group.

**9.** The method for generating singlet oxygen according to any one of claims 1 to 8, wherein the X-ray diffraction pattern has a plurality of peaks within a diffraction angle 2θ range of 5° to 35°, and full widths at half maximum of top two peaks with highest intensity within the range are not more than 0.70°.

**10.** The method for generating singlet oxygen according to any one of claims 1 to 9, wherein the phthalocyanine compound crystal is a crystal of a gallium phthalocyanine compound or a titanyl phthalocyanine compound.

**11.** The method for generating singlet oxygen according to any one of claims 1 to 10, wherein the phthalocyanine compound crystal contains a compound represented by a formula (1) below:

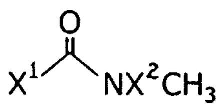 (1)

in formula (1), $X^1$ represents a hydrogen atom, a methyl group, or an ethyl group, and $X^2$ represents a hydrogen atom, a methyl group, or an ethyl group.

**12.** The method for generating singlet oxygen according to claim 11, wherein the phthalocyanine compound crystal contains a compound represented by a formula (2) below:

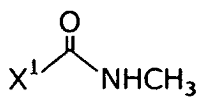 (2)

in formula (2), $X^1$ represents a hydrogen atom, a methyl group or an ethyl group.

**13.** The method for generating singlet oxygen according to claim 11 or 12, wherein the content of the compound represented by formula (1) in the phthalocyanine compound crystal is 0.10 to 1.70% by mass.

**14.** A system for generating singlet oxygen, the system comprising:

a phthalocyanine compound crystal; and
a device having a radiation source for irradiating the crystal with electromagnetic radiation, wherein
in an X-ray diffraction pattern of the phthalocyanine compound crystal obtained by X-ray diffraction measurement using CuKα rays, a peak is present within a diffraction angle 20 range of 5° to 35°, and a full width at half maximum of a peak with highest intensity within the range is not more than 2.50°, and
singlet oxygen is generated from the crystal.

**15.** The system for generating singlet oxygen according to claim 14, wherein a wavelength of the electromagnetic radiation is 0.001 to 10 nm.

**16.** A kit for generating singlet oxygen, the kit comprising:

a phthalocyanine compound crystal; and
a radiation source for irradiating the crystal with electromagnetic radiation, wherein
in an X-ray diffraction pattern of the phthalocyanine compound crystal obtained by X-ray diffraction measurement using CuKα rays, a peak is present within a diffraction angle 2θ range of 5° to 35°, and a full width at half maximum of a peak with highest intensity within the range is not more than 2.50°, and
singlet oxygen is generated from the crystal.

**17.** The kit for generating singlet oxygen according to claim 16, wherein a wavelength of the electromagnetic radiation is 0.001 to 10 nm.

**18.** An agent for generating singlet oxygen, the agent comprising

a phthalocyanine compound crystal, wherein
in an X-ray diffraction pattern of the phthalocyanine compound crystal obtained by X-ray diffraction measurement using CuKα rays, a peak is present within a diffraction angle 2θ range of 5° to 35°, and a full width at half maximum of a peak with highest intensity within the range is not more than 2.50°, and
singlet oxygen is generated by irradiating the crystal with electromagnetic radiation.

**19.** The agent for generating singlet oxygen according to claim 18, wherein a number-average particle diameter of the crystals is 30 to 300 nm.

**20.** The agent for generating singlet oxygen according to claim 18 or 19, wherein the phthalocyanine compound contains an atom with an atomic number of at least 11.

**21.** The agent for generating singlet oxygen according to any one of claims 18 to 20, wherein the phthalocyanine compound is a metal phthalocyanine compound having a central metal atom.

**22.** The agent for generating singlet oxygen according to claim 21, wherein the central metal atom is an atom with an atomic number of at least 11.

**23.** The agent for generating singlet oxygen according to claim 21 or 22, wherein the metal phthalocyanine compound has an axial ligand.

**24.** The agent for generating singlet oxygen according to claim 23, wherein the axial ligand is a halogen atom or a hydroxy group.

**25.** The agent for generating singlet oxygen according to any one of claims 18 to 24, wherein the X-ray diffraction pattern has a plurality of peaks within a diffraction angle 2θ range of 5° to 35°, and the full widths at half maximum of top two peaks with highest intensity within the range are not more than 0.70°.

**26.** The agent for generating singlet oxygen according to any one of claims 18 to 25, wherein the phthalocyanine compound crystal is a crystal of a gallium phthalocyanine compound or a titanyl phthalocyanine compound.

**27.** The agent for generating singlet oxygen according to any one of claims 18 to 26, wherein the phthalocyanine compound crystal contains a compound represented by a formula (1) below:

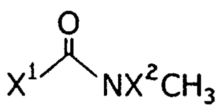 (1)

in formula (1), $X^1$ represents a hydrogen atom, a methyl group, or an ethyl group, and $X^2$ represents a hydrogen atom, a methyl group, or an ethyl group.

**28.** The agent for generating singlet oxygen according to claim 27, wherein the phthalocyanine compound crystal contains a compound represented by a formula (2) below:

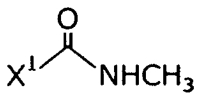 (2)

in formula (2), $X^1$ represents a hydrogen atom, a methyl group or an ethyl group.

**29.** The agent for generating singlet oxygen according to claim 27 or 28, wherein a content of the compound represented by formula (1) in the phthalocyanine compound crystal is 0.10 to 1.70% by mass.

**Amended claims under Art. 19.1 PCT**

**1.** A method for generating singlet oxygen,

the method including a step of generating singlet oxygen by irradiating a phthalocyanine compound crystal with electromagnetic radiation, wherein
in an X-ray diffraction pattern of the phthalocyanine compound crystal obtained by X-ray diffraction measurement using CuKα rays, a peak is present within a diffraction angle 2θ range of 5° to 35°,
a full width at half maximum of a peak with highest intensity within the range is not more than 2.50°, and
a number-average particle diameter of the crystals is 30 to 300 nm.

**2.** A method for generating singlet oxygen,

the method including a step of generating singlet oxygen by irradiating a phthalocyanine compound crystal with electromagnetic radiation, wherein
in an X-ray diffraction pattern of the phthalocyanine compound crystal obtained by X-ray diffraction measurement using CuKα rays, a peak is present within a diffraction angle 2θ range of 5° to 35°,
a full width at half maximum of a peak with highest intensity within the range is not more than 2.50°, and
a wavelength of the electromagnetic radiation is 0.001 to 10 nm.

**3.**

**4.** The method for generating singlet oxygen according to any one of claims 1, 2 and 4, wherein the phthalocyanine compound contains an atom with an atomic number of at least 11.

**5.** The method for generating singlet oxygen according to any one of claims 1, 2 and 4, wherein the phthalocyanine compound is a metal phthalocyanine compound having a central metal atom.

**6.** The method for generating singlet oxygen according to claim 5, wherein the central metal atom is an atom with an atomic number of at least 11.

**7.** The method for generating singlet oxygen according to claim 5 or 6, wherein the metal phthalocyanine compound has an axial ligand.

**8.** The method for generating singlet oxygen according to claim 7, wherein the axial ligand is a halogen atom or a hydroxy group.

**9.** The method for generating singlet oxygen according to any one of claims 1,2 and 4 to 8, wherein the X-ray diffraction pattern has a plurality of peaks within a diffraction angle 2θ range of 5° to 35°, and full widths at half maximum of top two peaks with highest intensity within the range are not more than 0.70°.

**10.** The method for generating singlet oxygen according to any one of claims 1, 2 and 4 to 9, wherein the phthalocyanine compound crystal is a crystal of a gallium phthalocyanine compound or a titanyl phthalocyanine compound.

**11.** The method for generating singlet oxygen according to any one of claims 1, 2 and 4 to 10, wherein the phthalocyanine compound crystal contains a compound represented by a formula (1) below:

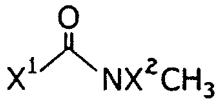 (1)

in formula (1), $X^1$ represents a hydrogen atom, a methyl group, or an ethyl group, and $X^2$ represents a hydrogen atom, a methyl group, or an ethyl group.

**12.** The method for generating singlet oxygen according to claim 11, wherein the phthalocyanine compound crystal contains a compound represented by a formula (2) below:

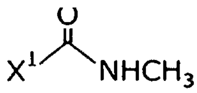 (2)

in formula (2), $X^1$ represents a hydrogen atom, a methyl group or an ethyl group.

**13.** The method for generating singlet oxygen according to claim 11 or 12, wherein the content of the compound represented by formula (1) in the phthalocyanine compound crystal is 0.10 to 1.70% by mass.

**14.** A system for generating singlet oxygen, the system comprising:

a phthalocyanine compound crystal; and
a device having a radiation source for irradiating the crystal with electromagnetic radiation, wherein
in an X-ray diffraction pattern of the phthalocyanine compound crystal obtained by X-ray diffraction measurement using CuKα rays, a peak is present within a diffraction angle 20 range of 5° to 35°, and a full width at half maximum of a peak with highest intensity within the range is not more than 2.50°,
a number-average particle diameter of the crystals is 30 to 300 nm, and
singlet oxygen is generated from the crystal.

**15.** A system for generating singlet oxygen, the system comprising:

a phthalocyanine compound crystal; and
a device having a radiation source for irradiating the crystal with electromagnetic radiation, wherein
in an X-ray diffraction pattern of the phthalocyanine compound crystal obtained by X-ray diffraction measurement using CuKα rays, a peak is present within a diffraction angle 20 range of 5° to 35°, and a full width at half maximum of a peak with highest intensity within the range is not more than 2.50°,
a wavelength of the electromagnetic radiation is 0.001 to 10 nm, and
singlet oxygen is generated from the crystal.

**16.** A kit for generating singlet oxygen, the kit comprising:

a phthalocyanine compound crystal; and
a radiation source for irradiating the crystal with electromagnetic radiation, wherein
in an X-ray diffraction pattern of the phthalocyanine compound crystal obtained by X-ray diffraction measurement using CuKα rays, a peak is present within a diffraction angle 20 range of 5° to 35°, and a full width at half maximum of a peak with highest intensity within the range is not more than 2.50°,
a number-average particle diameter of the crystals is 30 to 300 nm, and
singlet oxygen is generated from the crystal.

**17.** A kit for generating singlet oxygen, the kit comprising:

a phthalocyanine compound crystal; and
a radiation source for irradiating the crystal with electromagnetic radiation, wherein
in an X-ray diffraction pattern of the phthalocyanine compound crystal obtained by X-ray diffraction measurement using CuKα rays, a peak is present within a diffraction angle 20 range of 5° to 35°, and a full width at half maximum of a peak with highest intensity within the range is not more than 2.50°,
a wavelength of the electromagnetic radiation is 0.001 to 10 nm, and

singlet oxygen is generated from the crystal.

**18.** An agent for generating singlet oxygen, the agent comprising

a phthalocyanine compound crystal, wherein
in an X-ray diffraction pattern of the phthalocyanine compound crystal obtained by X-ray diffraction measurement using CuKα rays, a peak is present within a diffraction angle 2θ range of 5° to 35°, and a full width at half maximum of a peak with highest intensity within the range is not more than 2.50°,
a number-average particle diameter of the crystals is 30 to 300 nm, and
singlet oxygen is generated by irradiating the crystal with electromagnetic radiation.

**19.**

**20.** The agent for generating singlet oxygen according to claim 18, wherein the phthalocyanine compound contains an atom with an atomic number of at least 11.

**21.** The agent for generating singlet oxygen according to any one of claim 18 or 19, wherein the phthalocyanine compound is a metal phthalocyanine compound having a central metal atom.

**22.** The agent for generating singlet oxygen according to claim 21, wherein the central metal atom is an atom with an atomic number of at least 11.

**23.** The agent for generating singlet oxygen according to claim 21 or 22, wherein the metal phthalocyanine compound has an axial ligand.

**24.** The agent for generating singlet oxygen according to claim 23, wherein the axial ligand is a halogen atom or a hydroxy group.

**25.** The agent for generating singlet oxygen according to any one of claims 18 and 20 to 24, wherein the X-ray diffraction pattern has a plurality of peaks within a diffraction angle 2θ range of 5° to 35°, and the full widths at half maximum of top two peaks with highest intensity within the range are not more than 0.70°.

**26.** The agent for generating singlet oxygen according to any one of claims 18 and 20 to 25, wherein the phthalocyanine compound crystal is a crystal of a gallium phthalocyanine compound or a titanyl phthalocyanine compound.

**27.** The agent for generating singlet oxygen according to any one of claims 18 and 20 to 26, wherein the phthalocyanine compound crystal contains a compound represented by a formula (1) below:

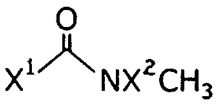 (1)

in formula (1), $X^1$ represents a hydrogen atom, a methyl group, or an ethyl group, and $X^2$ represents a hydrogen atom, a methyl group, or an ethyl group.

**28.** The agent for generating singlet oxygen according to claim 27, wherein the phthalocyanine compound crystal contains a compound represented by a formula (2) below:

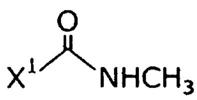 (2)

in formula (2), $X^1$ represents a hydrogen atom, a methyl group or an ethyl group.

**29.** The agent for generating singlet oxygen according to claim 27 or 28, wherein a content of the compound represented by formula (1) in the phthalocyanine compound crystal is 0.10 to 1.70% by mass.

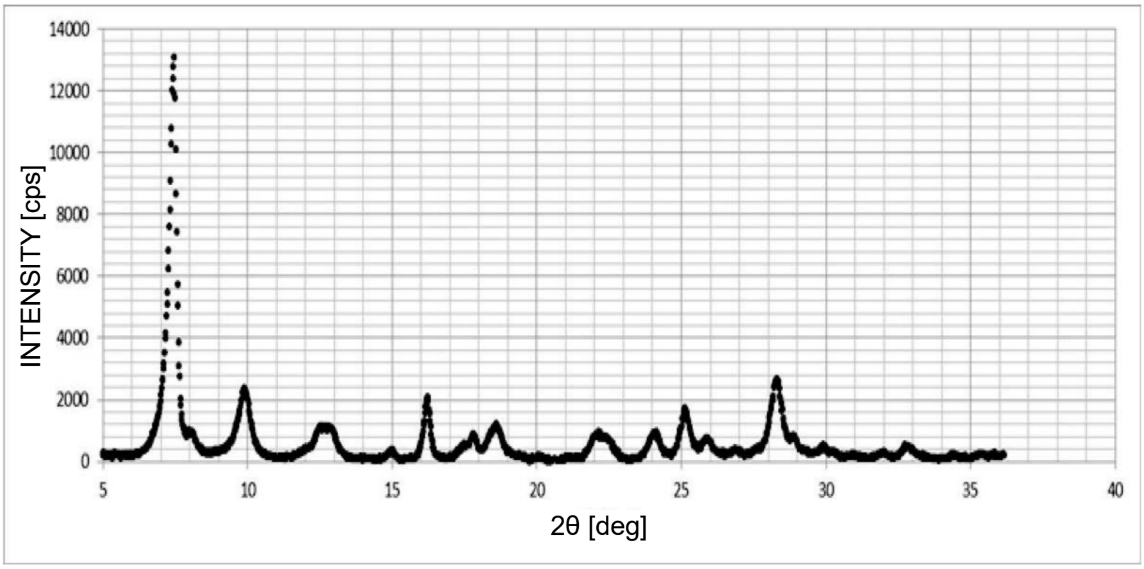
14000
12000
10000
8000
6000
4000
2000
0
INTENSITY [cps]
5
10
15
20
25
30
35
40
2θ [deg]

Fig. 1

# INTERNATIONAL SEARCH REPORT

International application No.
**PCT/JP2024/015705**

**A. CLASSIFICATION OF SUBJECT MATTER**

***C01B 13/02***(2006.01)i; ***A61K 33/00***(2006.01)i; ***A61P 31/04***(2006.01)i; ***A61P 31/12***(2006.01)i; ***A61P 35/00***(2006.01)i; *C07D 487/22*(2006.01)n
FI: C01B13/02 B; A61K33/00; A61P31/04; A61P31/12; A61P35/00; C07D487/22

According to International Patent Classification (IPC) or to both national classification and IPC

**B. FIELDS SEARCHED**

Minimum documentation searched (classification system followed by classification symbols)

C01B13/02; A61K31/00-33/44; A61N5/00-5/10; A61P31/04; A61P31/12; A61P35/00; C07D487/22

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JST7580/JSTChina (JDreamIII); CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| X | JP 2009-298970 A (FUJIFILM CORPORATION) 24 December 2009 (2009-12-24)<br>example 3, claims, paragraphs [0001]-[0019], [0143], fig. 7 | 1, 4-6, 9, 14, 16, 18, 20-22, 25 |
| X | KMENT, Stepan et al. Preparation of thin phthalocyanine layers and their structural and absorption properties. Thin Solid Films, 20 March 2009, vol. 517, no. 17, pp. 5274-5279, DOI: 10.1016/j.tsf.2009.03.067<br>abstract, p. 4479, right column second paragraph to p. 4480, right column first paragraph, p. 4485, left column, second paragraph to right column, first paragraph, fig. 1 | 1, 4-6, 9, 14, 16, 18, 20-22, 25 |
| X | PANICKER, Nisha S. et al. Effects of γ-ray irradiation and thermal annealing on structural, optical and electrical properties of vacuum deposited vanadyl 2, 3-naphthalocyanine thin films. Journal of Materials Science, 12 February 2011, vol. 46, no. 13, pp. 4479-4486, DOI: 10.1007/s10853-011-5341-y<br>abstract, p. 5274, left column, first paragraph to p. 5278, left column, first paragraph, p. 5279, right column, first paragraph, fig. 1, 4, tables 1, 2 | 1, 4-7, 9, 14, 16, 18, 20-23, 25 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **20 June 2024** | **02 July 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

**INTERNATIONAL SEARCH REPORT**

International application No.
**PCT/JP2024/015705**

**C. DOCUMENTS CONSIDERED TO BE RELEVANT**

| Category* | Citation of document, with indication, where appropriate, of the relevant passages | Relevant to claim No. |
|---|---|---|
| A | JP 2021-063036 A (TOYO INK SC HOLDINGS CO., LTD.) 22 April 2021 (2021-04-22) paragraphs [0007], [0065] | 1-29 |
| A | JP 2017-532340 A (THE UNIVERSITY OF CHICAGO) 02 November 2017 (2017-11-02) paragraph [0095] | 1-29 |
| A | CN 115645533 A (XIANGPENG YOUKANG (BEIJING) TECHNOLOGY CO., LTD.) 31 January 2023 (2023-01-31) paragraph [0003] | 1-29 |
| A | JP 06-056839 A (CIBA GEIGY AG) 01 March 1994 (1994-03-01) entire text | 1-29 |
| A | WO 2015/189980 A1 (CANON KABUSHIKI KAISHA) 17 December 2015 (2015-12-17) entire text, all drawings | 1-29 |

Form PCT/ISA/210 (second sheet) (July 2022)

**INTERNATIONAL SEARCH REPORT**
**Information on patent family members**

International application No.
**PCT/JP2024/015705**

| Patent document cited in search report | Publication date (day/month/year) | Patent family member(s) | Publication date (day/month/year) |
|---|---|---|---|
| JP 2009-298970 A | 24 December 2009 | (Family: none) | |
| JP 2021-063036 A | 22 April 2021 | (Family: none) | |
| JP 2017-532340 A | 02 November 2017 | US 2017/0231903 A1<br>paragraph [0187]<br>WO 2016/061256 A1<br>EP 3206987 A1<br>CN 107001031 A<br>MA 42161 A | |
| CN 115645533 A | 31 January 2023 | (Family: none) | |
| JP 06-056839 A | 01 March 1994 | US 5358940 A<br>entire text<br>EP 558449 A1<br>NZ 247000 A<br>AU 3376893 A<br>IL 104874 A<br>ZA 9301382 A<br>CA 2090772 A1<br>FI 930831 A<br>HU 64075 A<br>MX 9301083 A<br>SG 49615 A<br>KR 10-1993-0017912 A<br>CN 1075966 A | |
| WO 2015/189980 A1 | 17 December 2015 | US 2015/0362848 A1<br>entire text, all drawings<br>CN 106462090 A | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2009155239 A
- JP 2010523690 W
- JP 2017532340 W
- JP 2023071394 A
- JP 2024062199 A